# EUROPEAN PATENT APPLICATION

(11) **EP 1 491 891 A1**
(43) Date of publication of application: **29.12.2004**
(21) Application number: 03715643.7
(22) Date of filing: 31.03.2003
(51) Int. Cl.: G01N 33/543, G01N 33/48, G01N 33/72

(54) **BLOOD PROCESSING METHOD, BLOOD PROCESSING DEVICE, METHOD OF MEASURING HEMOGLOBINS AND DEVICE FOR MEASURING HEMOGLOBINS**

(30) Priority: 29.03.2002 JP 2002097514
(71) Applicant: MATSUSHITA ELECTRIC INDUSTRIAL CO., LTD., Kadoma-shi, Osaka 571-8501 (JP)
(72) Inventor: KITAWAKI, Fumihisa, Katano-shi, Osaka 576-0021 (JP); SHIGETOH, Nobuyuki, Kyotanabe-shi, Kyoto 610-0354 (JP); KAWAMURA, Tatsurou, Kyotanabe-shi, Kyoto 610-0351 (JP)
(74) Representative: Grünecker, Kinkeldey, Stockmair & Schwanhäusser Anwaltssozietät
(86) International application number: PCT/JP2003/004065
(87) International publication number: WO 2003/083479

(57) **Abstract**

A blood processing method according to the present invention includes Step St1 of preparing a blood specimen sample and a hemolyzing agent, and Step St2 of mixing the blood specimen sample and the hemolyzing agent to prepare a mixture sample. In this case, the hemolyzing agent is a solid reagent for destroying erythrocyte membranes.

## Description

### TECHNICAL FIELD

The present invention relates to blood processing methods for destroying erythrocyte membranes and measurement methods for a measuring hemoglobin.

### BACKGROUND ART

Presently, in the field of clinical examination, point of care tests (which will be herein referred to as POCTs) have attracted attention. The term "POCTs" means laboratory tests in medical scenes, and more specifically, is used as a general term for tests in which a time from collecting a sample to obtaining a test result is regarded as one of the most important factors. Therefore, in the field of clinical examination, simple operation, fast determination, and low costs are required for POCTs, in general. These conditions are also required for measurements of blood components.

Blood components can be largely divided into cell components and liquid components. Cell components of blood includes erythrocytes, leukocytes, and platelets. Liquid components of blood include plasma.

In a blood specimen sample, the volume percent of erythrocytes of whole blood (which will be herein referred to as a hematocrit value or a Ht value) is one of important factors. The Ht value varies according to the degree of anemia and is used as a measure of the degree of anemia. Moreover, since physical properties of the blood specimen sample (e.g., viscosity) influence measurements, the Ht value can be a measure of necessity for collections of other measurement values.

About 1.1 x 10¹⁰ erythrocytes exist in a cubic volume of 1 ml, and each erythrocyte has a volume of about 90 µm³. Water, which is the maximum component of an erythrocyte, has about 63 g in 100 ml of erythrocytes. Most of the about 34 g remaining of the erythrocyte can be considered to be hemoglobins. When the Ht value is 45%, hemoglobins have about 150 g per 1 L. More specifically, hemoglobins exist in blood at a concentration of 150 g/ l, and are of a type of protein exiting in the largest quantity in a living organism.

Human hemoglobin (which will be herein referred to as "Hb") is an associated tetramer in which each of α chain and non-α chain (β, γ and δ chains) globins is bound to a heme. Hb in a healthy person contains about 90% of HbA (α₂β₂), about 3% of HbA₂ (α₂δ₂), about 1% of HbF (α₂γ₂) and several % of HbA₁ (HbA to which sugar binds). Note that HbA₁ described above includes HbA₁ₐ, HbA_{1b}, and HbA_{1c}.

Conventionally, it has been considered important in view of clinical chemistry to examine the composition of the above-described Hb. For example, if a measurement value for the HbF is an abnormal level which is out of a reference value range from 0.3% to 1.3%, hereditary persistence of fetal hemoglobinemia, chronic myeloid leukemia, or acute myeloid leukemia is suspected. Moreover, if a measurement value for the HbA₂ is an abnormal level which is out of a reference value range from 2% to 3.5%, unstable Hb syndrome or pernicious anemia is suspected.

Furthermore, in recent years, diabetes, as one of three major geriatric diseases, has become a serious problem. Then, HbA₁ or HbA_{1c} has attracted attention as an item to be examined in order to give a standard for a relatively long-term blood sugar control over 1-3 months for patients with diabetes. Measurement apparatuses for measuring HbA_{1c} have been developed.

A hemoglobin is measured using, for example, an isoelectric focusing method, ion exchange chromatgraphy, latex immunoagglutination or like methods.

Unlike other subject substances to be measured in a blood specimen sample, hemoglobins are present inside of erythrocytes. Therefore, hemolysis has to be induced for performing measurement. "Hemolysis" is the phenomenon in which erythrocyte membranes are destroyed and hemoglobins are eluted out of erythrocytes. The size of erythrocytes is influenced by osmotic pressure against erythrocyte membranes. In a salt solution whose salt concentration is higher than that of a saline solution (0.9% NaCl), erythrocytes shrink. On the other hand, in a salt solution whose salt concentration is lower than that of a saline solution (0.9% NaCl), erythrocytes swell. If erythrocyte membranes are destroyed during shrinkage or swelling of erythrocytes, hemoglobins are eluted out of erythrocytes. Assume that blood is completely hemolyzed. When the Ht value is 45%, the concentration of hemoglobins is about 150 g/L.

### Problems to be solved

A measurement result for a hemoglobin is expressed by the percentage of the amount of a measured hemoglobin to the amount of the total hemoglobins. Therefore, not only the amount of a hemoglobin to be measured but also the amount of the total hemoglobins has to be measured. As has been described, although there are individual differences, the amount of the total hemoglobins in blood is normally about 150 g/L. This is a very high concentration level, which is inconvenient in measuring a hemoglobin. A hemoglobins has a red pigment having an absorption wavelength in the longer wavelength side. Therefore, measurement of a hemoglobin can be easily hindered at a high concentration.

For example, when a hemoglobin is measured with immunochromatography, as a characteristic of an immunoreaction, a measurement value in an antigen excess zone is reduced. This reduction might cause misoperation. Moreover, because of increase in viscosity of a blood specimen sample caused by the presence of a large amount of hemoglobins, the speed of development of the blood specimen sample with a solid phase of an immunochromatography device is reduced, the blood specimen sample is not developed at all, or other inconveniences occur. Therefore, as disclosed in Japanese Unexamined Patent Publication No. 3-46566, many operations for diluting a blood specimen sample have to be performed after hemolysis operation in order to conduct precise measurement of a hemoglobin. That is to say, to measure a hemoglobin, many operations are required for processing a blood specimen sample.

The present invention has been devised in view of the above-described problems, and relates to blood processing methods and devices, and measurement methods and devices for measuring a hemoglobin. More specifically, it is an object of the present invention to provide method and device which allow measurements of components in erythrocytes, represented by hemoglobins, to be performed in a more simple manner.

### DISCLOSURE OF INVENTION

A blood processing method according to the present invention includes the steps of: (a) preparing a blood specimen sample containing erythrocytes, and a hemolyzing agent; and (b) mixing the blood specimen sample and the hemolyzing agent to prepare a mixture sample, and the hemolyzing agent is a solid reagent for destroying erythrocyte membranes.

In a known method, many operations for processing a blood sample are required in order to measure components in erythrocytes, represented by hemoglobins. However, with the blood processing method of this embodiment, many of operations for diluting a blood specimen sample after hemolysis operation are not necessary in measuring components in erythrocytes. Therefore, with the blood processing method of this embodiment, components in erythrocytes can be measured in a more simple manner.

It is preferable that in the step (b), hemocyte components remain in the mixture sample.

The blood processing measure may be configured so that the hemolyzing agent is a reagent for changing osmotic pressure against erythrocyte membranes.

The blood processing measure may be configured so that the hemolyzing agent is an inorganic salt.

It is preferable that the concentration of the inorganic salt in the mixture sample is in a range of 0.05-10% (w/v).

A hemoglobin measurement method according to the present invention includes the steps of: (a) preparing a blood specimen sample containing erythrocytes, and a hemolyzing agent; (b) mixing the blood specimen sample and the hemolyzing agent to prepare a mixture sample; and (c) measuring a hemoglobin contained in plasma in the mixture sample, and the hemolyzing agent is a solid reagent for destroying erythrocyte membranes.

In a known method, many operations for processing a blood sample are required in order to measure components in erythrocytes, represented by hemoglobins. However, with the blood processing method of this embodiment, many of operations for diluting a blood specimen sample after hemolysis operation are not necessary in measuring components in erythrocytes. Therefore, with the blood processing method of this embodiment, components in erythrocytes can be measured a more simple manner.

The hemoglobin measurement method may be configured so that in the step (c), as the hemoglobin measurement method for measuring a hemoglobin contained in plasma in the mixture sample, any one of an immunochromatography, an immunoconcentration assay and a solid phase enzyme linked immunoassay, a colorimetry slide and an electrode method slide is used.

The hemoglobin measurement method may be configured so that in the step (c), at least two hemoglobins are measured.

The hemoglobin measurement method may be configured so that said at least two hemoglobins are total hemoglobins and HbA1c.

The hemoglobin measurement method may be configured so that the hemoglobin is a glycated hemoglobin.

A blood processing device according to the present invention includes: a base material; and a solid hemolyzing agent for destroying erythrocyte membranes, and the hemolyzing agent is carried on the base material at an amount at which when a mixture sample of a blood specimen sample containing erythrocytes and the hemolyzing agent is formed, hemocyte components remain in the mixture sample.

With the blood processing device, measurements of components in erythrocytes can be performed in a more simple manner.

A hemoglobin measurement device according to the present invention is a hemoglobin measurement device for measuring a hemoglobin in a blood specimen sample containing erythrocytes, wherein the hemoglobin measurement device includes: a base material having a sample adding portion to which the blood specimen sample is added; a hemolyzing agent for destroying erythrocyte membranes; a detection substance capable of specifically binding to a hemoglobin; and an immobilization substance capable of specifically binding to the hemoglobin, the hemolyzing agent is carried on the base material at an amount at which when a mixture sample of a blood specimen sample containing erythrocytes and the hemolyzing agent is formed, hemocyte components remain in the mixture sample, the hemolyzing agent, the detection substance and the immobilization substance are individually carried in a solid form on the base material, and the hemolyzing agent, the detection substance and the immobilization substance are arranged in this order from part of the base material closer to a sample adding portion to which the blood specimen sample is added.

With the hemoglobin measurement device of the present invention, measurement of a hemoglobin can be performed in a more simple manner.

Another hemoglobin measurement device according to the present invention is a hemoglobin measurement device for measuring a hemoglobin in a blood specimen sample containing erythrocytes, wherein the hemoglobin measurement device includes: a substrate; a sample adding portion which is provided on the base material and to which the blood specimen sample is added; a hemolyzing layer which is provided on the base material, contains a solid hemolyzing agent for destroying erythrocyte membranes, and is for controlling hemolysis of the blood specimen sample; a labeling layer which is provided on the base material, contains a detection substance capable of specifically binding to a hemoglobin, and is for labeling the hemoglobin; and a detecting layer which is provided on the base material, contains an immobilization reagent capable of specifically binding to a hemoglobin, and is for detecting the hemoglobin, and the blood specimen sample can move between the hemolyzing layer, the labeling layer and the detecting layer.

With the hemoglobin measurement device, measurement of a heoglobin can be performed in a more simple manner.

The hemoglobin measurement device may be configured so that the hemoglobin measurement device further includes a layer for removing remaining erythrocyte components after the blood specimen sample has been hemolyzed.

The hemoglobin measurement device may be configured so that the hemolyzing layer, the labeling layer and the detecting layer are arranged in a single layer.

### BRIEF DESCRIPTION OF DRAWINGS

FIG. **1** is a flowchart of a blood processing method according to the present invention.
FIG. **2** is an equation of hemolysis percentage.
FIGS. **3(a)** through **3(b)** are illustrations of a blood processing device according to an embodiment of the prsent invention.
FIG. **4** is an illustration of an exemplary hemoglobin measurement device of a single-layer type according to the present invention.
FIG. **5** is a cross-sectional view of an exemplary hemoglobin measurement device of a multilayer type according to the present invention.
FIG. **6** is a graph of KCl concentration (concentration of added hemolyzing agent) and light transmittance in an example of the present invention.
FIG. **7** is a graph showing relationships between KCl concentration (concentration of added hemolyzing agent) and Hb concentration, and HbA1c in an example of the present invention.
FIG. **8** is a graph showing relationship between KCl concentration (concentration of added hemolyzing agent) and reflection absorbance at a wavelength of 610 nm.
FIG. **9** is a graph showing standard curve for Hb measurement device in an example of the present invention.
FIG. **10** is a graph showing standard curve for HbA_{1c} measurement device in an example of the present invention.
FIG. **11** is a graph showing measurement results obtained by performing measurements for 4.0% HbA1c, 5.4% HbA_{1c}, and 10.4 HbA_{1c}.
FIG. **12** is a graph showing calculation results obtained by calculating concentrations, using standard curves, from values measured for 4.0% HbA1c, 5.4% HbA_{1c}, and 10.4 HbA_{1c} by a device in an example of the present invention.

### BEST MODE FOR CARRYING OUT THE INVENTION

Hereinafter, embodiments of the present invention will be described with reference to the accompanying drawings.

### (EMBODIMENT 1)

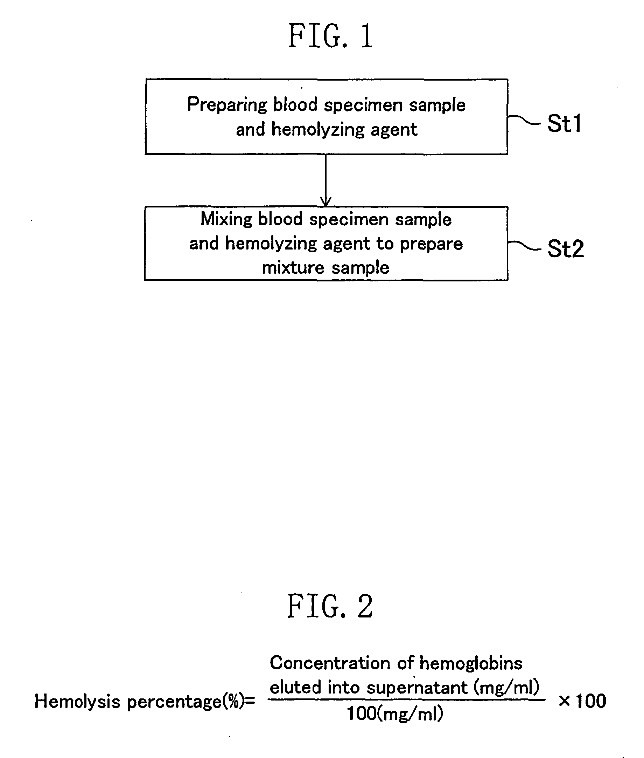

In this embodiment, a blood processing method according to the present invention will be described with reference to the accompanying drawings. FIG. **1** is a flowchart of the blood processing method of the present invention.

As shown in FIG. **1**, the blood processing method of this embodiment includes Step St1 of preparing a blood specimen sample and a hemolyzing agent and Step St2 of mixing the blood specimen sample and the hemolyzing agent to prepare a mixture sample. In this case, the hemolyzing agent is a solid agent for destroying erythrocytes.

The "blood specimen sample" is blood collected from a specimen such as a human using a regular blood collection method (e.g., with intravenous injection or Lancet).

As the hemolyzing agent, a solid agent for destroying erythrocyte membranes is used. For example, it is preferable that the hemolyzing agent is prepared in a powder form by freeze-drying so that the hemolyzing agent contains almost no moisture.

Moreover, the blood processing method of this embodiment is preferably used in measurement of a hemoglobin. That is to say, a hemoglobin measurement method according to the present invention includes Step St1 of preparing a blood specimen sample and a hemolyzing agent and Step St2 of mixing the blood specimen sample and the hemolyzing agent to prepare a mixture sample, which are shown in FIG. **1**, and in addition to Steps St1 and St2, further includes the process step of measuring a hemoglobin contained in plasma in the mixture sample. In this case, the hemolyzing agent is a solid agent for destroying erythrocyte membranes.

According to the blood processing method of this embodiment, a hemoglobin contained in plasma in the mixture sample can be quantitatively measured with a method known by persons skilled in the art.

As shown in FIG. **1**, in Step St2, when a mixture sample is prepared by mixing the blood specimen sample and the hemolyzing agent, erythrocyte membranes of erythrocytes in the blood specimen sample are destroyed, so that components (typically hemoglobins) in the erythrocytes are eluted out of the erythrocytes (i.e., hemolysis ocuurs). Thus, components (typically, hemoglobins) in the erythrocytes can be measured.

As has been described, many operations for processing a blood sample are required in order to measure components in erythrocytes, represented by hemoglobins. However, with the blood processing method of this embodiment, many of operations for diluting a blood specimen sample after hemolysis operation are not necessary in measuring components in erythrocytes. Therefore, with the blood processing method of this embodiment, components in erythrocytes can be measured in a more simple manner.

Assume that a hemoglobin is measured using an immunological method. With complete hemolysis, the hemoglobin concentration in a sample becomes very high and thus the sample is considered to be in an antigen excess zone, so that sensitivity in measurement might be reduced. Therefore, in Step St2, it is preferable to create a state where hemocyte components remain in a mixture sample, i.e, to partially hemolyze a blood specimen sample.

To "partially hemolyze a blood specimen sample" means to hemolyze a less volume of erythrochytes than that in complete hemolysis. For example, in the case of blood having a Ht value of 45% and a volume of 1 L, less than 450 ml erythrocytes are hemolyzed. In contrast, "complete hemolysis" means a state in which substantially the total volume of erythrochytes has been hemolyzed. For example, in the case of blood having a Ht value of 45% and a volume of 1 L, about 450 ml erythrocytes are hemolyzed. By controlling a blood specimen sample to partially hemolyze it, membranes of part of erythrocytes in the sample are destroyed, so that hemoglobins therein can be hemolyzed. In this case, the concentration of hemoglobins in the plasma is lower than that in complete hemolysis. For example, when erythrocytes corresponding to a volume of 1 ml are hemolyzed in blood having a Ht value of 45% and a volume of 1 L, 0.34 g hemoglobins and 0.63 g water are eluted in 550.63 ml plasma. Accordingly, 0.34 g hemoglobins are dissolved in 550.63 plasma and the hemoglobin concentration is 0.62 g/L. The concentration of hemoglobins in a complete hemolyzed state is 150 g/L. In the blood processing method of this embodiment, a buffer solution or the like is not necessary, and the same effects as those in diluting blood to 250% of the volume of complete hemolysis can be achieved only by performing hemolysis.

As has been described in BACKGROUND ART, a Ht value is the volume percent of erythrocytes to whole blood. Therefore, the amount of erythrocytes and the amount of total hemoglobins can be understood by a Ht value. Note that a Ht value is determined by a method known by persons skilled in the art, such as a capillary method and the Wintrobe method. When a Ht value is determined by a capillary method, the Ht value can be 36-48% in male adults and 34-43% in female adults. For infants, the Ht value can be 44-64% in newborns (umbilical cord blood), 32-44% in infants 3 months of age, 36-44% in infants 1 year of age, and 37-44% in children 10-12 years of age (See Dacie et al., *Rinshou Kensahou Teiyou (Clinical Examination Method) 31*^{*st*} *edition,* Kanehara Co., Ltd.). Thus, a Ht value is determined for hemoglobin measurement, taking sexes and ages of specimens into consideration.

As for the concentration of the total hemoglobins, although there are individual differences in the Ht value or the like, most people have more than 100 mg/ml of Hb (males: about 130-170 mg/l, females: about 120-150 mg/l). With a known measurement apparatus, Hb at a higher concentration than 100 mg/ml can not be measured without performing dilution operation. Therefore, the Hb concentration obtained by "partial hemolysis" is preferably 100 mg/ml or less.

Control of hemolysis can be performed, for example, by changing the amount of a hemolyzing agent. For example, when 1.5 ml of blood is added to 1.5 mg of potassium chloride and 7.5 mg of potassium chloride, the concentrations of the total hemoglobins in the former case and the latter case are about 6.0 g/L and about 25 g/L, respectively. In this manner, as the amount of a hemolyzing agent is larger, the hemolysis percentage becomes larger. Then, how to determine the amount of a hemolyzing agent with which an optimum hemoglobin concentration in plasma (optimum hymolysis percentage) is achieved will be described.

First, a blood specimen sample is hemolyzed with an arbitrary amount of a hemolyzing agent and then separation is performed to obtain a supernatant fluid. Next, the hemoglobin concentration in the supernatant fluid is measured. Subsequently, the relationship between the amount of the added hemolyzing agent and the hemoglobin concentration is determined. Finally, an amount of the hemolyzing agent to be added, with which an optimum hemoglobin concentration in plasma is achieved, is determined based on the relationship determined above.

More specifically, the amount of the hemolyzing agent with which an optimum hemoglobin concentration in plasma is determined by obtaining a relationship diagram between the amount of the added hemolyzing agent and the hemoglobin concentration according to almost the same procedure as that in Example 2 described later.

The optimum hemoglobin concentration in plasma (optimum hemolysis percentage) depends on a measurement method which is used. Accordingly, the amount of the hemolyzing agent to be added also depends on the measurement method. Note that in this application, the hemolysis percentage is expressed by the equation shown in FIG. **2**.

Assume that the Ht value is 40-50%. If absorptiometry is used as a measurement method for measuring hemoglobins, a hemolyzing agent is preferably added at an amount at which the hemolysis percentage is 0.01-1% (hemoglobin concentration is 0.01-1 mg/ml). If immunochromatography is used, a hemolyzing agent is preferably added at an amount at which the hemolysis percentage is 0.0001-0.1% (hemoglobin concentration is 0.1-10 mg/dl).

When a measurement is performed by an optical asborbance method, the maximum molar extinction coefficient of Hb is about 10⁵M⁻¹. Therefore, the amount of the hemolysis agent to be added is preferably an amount at which the hemoglobin concentration is 0.1-1 mg/ml (the concentration with which absorbance is about 0.1-2).

When a measurement is performed by immunochromatography, the amount of the hemolysis agent to be added is determined based on the hemoglobin concentration depending on characteristics (e.g., binding affinity) of an antibody used in immunochromatography.

For example, if potassium chloride is used as a hemolyzing agent, the percentage (concentration) of the added hemolyzing agent to the total volume of a blood specimen sample is preferably in a range of 0.05-10% (w/v) (the unit for (w/v) is assumed to be g/ml in this application). Specifically, in a measurement by absorptiometry, the percentage (concentration) of the added hemolyzing agent to the total volume of a blood specimen sample is preferably in a range of 0.05-1% (w/v). In a measurement by immunochromatography, the percentage (concentration) of the added hemolyzing agent to the total volume of a blood specimen sample is preferably in a range of 0.05-3% (w/v), although it depends to some extent on the size of a chromatography device, the amounts of an immobilized antibody and a reagent, i.e., an labeled antibody, or the like. More specifically, if a chromatography device having a size of 5 mm x 10 cm is used, the percentage (concentration) of the added hemolyzing agent to the total volume of a blood specimen sample is preferably in a range of 0.1-1% (w/v).

If some other salt is used, the salt may be added so that the concentration of the salt is equivalent to that of potassium chloride. Moreover, for example, a surfactant such as lauric acid sodium or a reagent which directly influences erythrocyte membranes such as an anti-erythrocyte antibody can be also used. In such a case, after having determined the necessary amount of the hemolyzing agent for achieving an optimum hemoglobin concentration in plasma using the total hemoglobins concentration measurement method described above, a blood specimen sample may be partially hemolyzed with the determined amount of the hemolyzing agent.

Once the necessary amount of the hemolyzing agent for achieving the optimum hemoglobin concentration in plasma has been determined, the necessary amount of the hemolyzing agent is used to control hemolysis, so that the blood specimen sample is partially hemolyzed. Subsequently, an arbitrary hemoglobinometry presently used in clinical examinations is used to measure the concentration of the total hemoglobins in the blood specimen sample. Methods of hemoglobinometry includes, for example, a cyanmethemoglobin method, an oxihemoglobin method and an SLS-hemoglobin method (for example, see *Rinshou Kensahou Teiyou (Clinical Examination Method) 31*^{*st*} *edition,* Kanehara Co., Ltd.). Ex vivo diagnostic agents specially used in the hemoglobinometry methods are commercially available.

According to the blood processing method and hemoglobin measurement method of this embodiment, in measuring a hemoglobin, it is possible to omit many operations for diluting a blood specimen sample after hemolysis operation, which have been required in a known method, so that an appropriate hemoglobin concentration for measurement can be achieved in a more simple manner.

In this embodiment, as the hemolyzing agent, a reagent for changing osmotic pressure against erythrocyte membranes is used. Erythrocytes shrink in a salt solution at a higher salt concentration than that of a saline solution (0.9% NaCl). On the other hand, in a salt solution at a lower salt concentration than that of the saline solution (0.9% NaCl), erythrocytes swell. Due to the shrinkage and swelling of erythrocytes, erythrocyte membranes are destroyed, so that hemoglobins are eluted out of erythrocytes. For example, an inorganic salt (e.g., potassium chloride, sodium chloride, or sodium fluoride) is used. More specifically, potassium chloride is preferable. As has been described, hemolysis occurs when erythrocytes are placed in an environment other than in a saline solution (0.9% NaCl). Therefore, the reagent is added to the blood specimen sample at a concentration or an amount at which the salt concentration in blood is under the above-described condition (i.e., in an environment other than 0.9% NaCl and a salt concentration of equivalent to 0.9% NaCl) and at which a desired amount of hemoglobins is eluted into plasma. For example, if 250 µg of potassium sodium is used with 50 µl of blood, the hemolysis percent is about 2%.

Note that the hemolyzing agent is used in a solid form. As for the hemolyzing agent, a hemolyzing agent prepared in the manner in which a solution containing a desired amount of a hemolytic reagent is prepared and then freeze-dried may be used. Moreover, for example, a hemolyzing agent carried by a base material (such as a membrane), which has been obtained by soaking the base material with the solution containing the desired amount of the hemolytic reagent and freeze-drying it, may be used.

The blood specimen sample is processed with the hemolysis agent before measurement of a hemoglobin are started. Alternatively, the blood specimen sample is processed with the hemolysis agent simultaneously with measurement of a hemoglobin. In either case, hemoglobins eluted out due to hemolysis are measured by the following means or technology available in the art. As an embodiment of such a method, when the hemolyzing agent is in a liquid form, the hemolyzing agent may be added to a blood specimen sample before a hemoglobin in the blood specimen sample is measured. If the hemolyzing agent is in a solid form and carried by a base material, a blood specimen sample may be supplied to measurement means after being introduced to the base material, or the sample may be introduced to the base material when the base material is supplied to the measurement means.

Hemoglobins to be measured include Hb, HbA(α₂β₂), HbA₂(α₂δ₂), HbF(α₂γ₂) and HbA₁ (a bound body of HbA and sugar), and also HbA₁ₐ, HbA_{1b} and HbA_{1c} into which HbA₁ is further divided. Herein, Hb comprehensively means to be total hemoglobins including the above-described hemoglobins. HbA_{1c}, and HbA₁ₐ, HbA_{1b} and HbA_{1c} into which HbA_{1c}, is further divided are comprehensively called glycohemoglobin or "glycated hemoglobin" and have the same primary structure. In each glycated hemoglobin, an amino group of N-terminal valine of a β-chain is blocked by sugar or a derivative of sugar. The term "glycated hemoglobin" used in this application includes HbA₁, and HbA₁ₐ, HbA_{1b} and HbA_{1c} into which HbA_{1,} is further divided. More specifically, HbA_{1c} is a glycated hemoglobin to which glucose is nonenzymatically bound.

It is important in view of clinical chemistry to examine a Hb composition. To achieve clinical significance, at least two hemoglobins in a blood specimen sample processed in the above-described manner may be measured.

In this embodiment, specifically, by controlling partial hemolysis, the Hb (total hemoglobins) concentration in a blood specimen sample can be suppressed to a low level. Thus, a measured hemoglobin concentration is not an absolute value. That is to say, measurement values for HbA_{1c} obtained by binding of sugar and HbA, HbA₂, HbF or HbA, and HbA₁ₐ, HbA_{1b} and HbA_{1c} into which HbA_{1c}, is further divided, or the like are expressed by their percentages to the total Hb amount. Then, each of these percentages to the total Hb amount may be a subject for measurement in the present invention because they are not varied according to a hemolysis percentage. Moreover, an absolute value for the hemoglobin concentration is in a proportional relationship to a hemolysis percentage. Therefore, it is possible to calculate the hemoglobin concentration backward from a measurement value.

Glycated hemoglobins (specifically, HbA₁ or HbA_{1c}) have attracted attention as an index of blood sugar control for patients with diabetes, and more specifically, as an index of a long term (mainly 1-3 months) blood sugar control for patients with diabetes. For example, normally, HbA₁ is 7-8% of Hb (total hemoglobins) and HbA_{1c} is 6% or less (e.g., 4.7-5.7%) of Hb (total hemoglobins). However, these glycated hemoglobins are increased in patients with diabetes. For example, HbA_{1c} accounts for 20% of Hb. Therefore, by using the measurement method of this embodiment as a method for measuring the glycated hemoglobins, the measurement method of this embodiment can be preferably applied to diagnosis and treatment and the like of diabetes. Moreover, it is also possible to measure Hb (total hemoglobins) and HbA_{1c} with the measurement method of the present invention.

Moreover, according to the measurement method of this embodiment, HbF can be measured as the percentage thereof to Hb. The percentage of HbF to Hb is 0.3-1.3% in healthy adults. The percentage of HbF to Hb is larger than this reference range in cases of hereditary persistence of fetal hemoglobinemia, chronic myeloid leukemia, Fanconi anemia, erythroleukemia, paroxysmal nocturnal hemoglobinuria, refractory anemia, pregnancy, hydatidiform mole, thalassemia (β, δβ), and unstable hemoglobinemia. The percentage of HbF to Hb is larger than the reference range in rare cases of aplastic anemia, leukemia, plethora (polycythemia), myelofibrosis, malignant tumor and thyrotoxicosis. Therefore, the measurement method of this embodiment can be used for diagnosis of the diseases described above.

Furthermore, according to the measurement method of this embodiment, HbA₂ can be measured as a percentage to Hb. The percentage of HbA₂ to Hb is 2-3.5% in healthy people. The percentage of HbA₂ to Hb is higher than this reference range in cases of β thalassemia, unstable hemoglobinemia, sickle cell anemia (hetero type), megaloblastic anemia, and hyperthyroidism. On the other hand, the percentage of HbA₂ to Hb is lower than the reference range in the cases of α-, δ- or δβ-thalassemia, hereditary persistence of fetal hemoglobinemia, sideropenic anemia, and sideroblastic anemia. Therefore, the measurement method of this embodiment can be used for diagnosis of the diseases described above.

The Hb components described above have different isoelectric points, and thus may be quantitatively fractionated by ion exchange chromatography. Ion exchange chromatography may be performed using a column (product name: PolyCAT A™) commercially available from CYPRESS International Ltd. However, any commercially available columns having the same fractionation function as that of this column may be used. The Hb components described above may be measured by an immunological method using antibodies which are capable of recognizing the components, respectively. The immunological method include, for example, immunochromatography, immunoconcentration assay, solid phase enzyme linked immunoassay (ELISA assay) and latex immunoagglutination.

The concentration of the total hemoglobins can be measured using an arbitrary hemoglobinometry presently used in clinical examination, as has been described. Methods for such hemoglobinometry includes, for example, a cyanmethemoglobin method, an oxyhemoglobin method and a SLS-hemoglobin method can be used (for example, see *Rinshou Kensahou Teiyou (Clinical Examination Method) 31*^{*st*} *edition,* Kanehara Co., Ltd.). Ex vivo diagnostic agents used in the hemoglobinometry methods are commercially available.

Moreover, hemoglobins are measured by a measurement method using dry chemistry. The "measurement method by dry chemistry" is a measurement method for a subject substance in a sample in the following manner. The sample in a liquid form is added (normally, dripped) to a solid phase matrix in which a reagent is carried in a dry state so that the sample reacts with the reagent on the matrix, and then the reaction is detected. The measurement method by dry chemistry is a fast, simple and highly accurate measurement method. Therefore, the measurement method is conveniently used in routine clinical examination. The measurement method by dry chemistry includes, for example, an immunological method such as an immunochromatography, an immunoconcentration assay and a solid phase enzyme linked immunoassay, a colorimetry slide and an electrode method slide. Dry chemistry is disclosed in, for example, *RinshouByori: Dry Chemistry*/*Kan'ikensa no Aratanaru Tenkai (Clinicopathology: Dry Chemistry*/ *New Development of Simplified Examination)* (Japanese Society of Laboratory Medicine, November 1997).

### (EMBODIMENT 2)

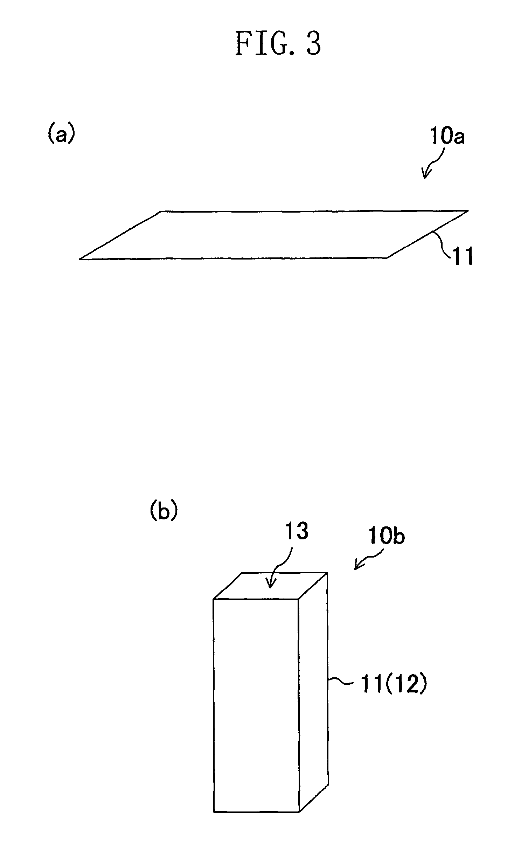

In this embodiment, a blood processing device which can be used in the blood processing method and hemoglobin measurement method described in EMBODIMENT 1 will be described with reference to the accompanying drawings. FIGS. **3(a)** and **3(b)** are illustrations of a blood processing device according to this embodiment.

As shown in FIG. **3(a),** a blood processing device **10a** according to this embodiment includes a base material **11** to which a blood specimen sample is to be supplied and a hemolyzing agent (not shown) at an amount at which a blood specimen sample is partially hemolyzed. The hemolyzing agent is carried by the base material **11**. Note that the hemolyzing agent is a solid reagent which destroys erythrocyte membranes. The hemolyzing agent is first added to the base material in a liquid form and then freeze-dried, and is carried by the base material in a dry state. Preferably, the hemolyzing agent is carried in a dry state in a region of the base material in which the blood specimen sample can be in contact with the hemolyzing agent.

As the base material **11** to which the blood specimen sample is to be supplied, there are various types of base materials raging from plate shape types to which a blood specimen sample is dripped (e.g., a insoluble, singly-layer base material such as Seralyzer (Ames), a test paper and an immunochromatography (plastic card type), or a multilayer base material such as Ektachem (Kodak) and DRI-CHEM (Fuji Film) ) to vessel shape types into which a liquid sample is put (e.g., a vial, a test tube, an ampule, a SHINO-TEST, a centrifuge rotor, and a piccoplo). A base material which can be used in the device of this embodiment is made of a material such as a paper towel (e.g., a pulp nonwoven fabric such as Reed Cooking Paper™), a mesh cloth (e.g., a gauze cotton cloth) and a glassfiber filter paper, which can absorb a liquid to store it.

As means for measuring a hemoglobin based on ion exchange chromatography or an immunological method (e.g., latex immunoagglutination) is used.

Moreover, instead of the blood processing device **10a** shown in FIG. **3(a),** a blood processing device **10b** shown in FIG. **3(b)** can be used. The base material **11** used in the blood processing device **10b** is a cell **12** which includes side faces, a bottom face (not shown) and an opening **13** and is suitable for use as measurement means. In this case, a hemolyzing agent is carried in a dry state in a region of a cell (e.g., the inner surface of the bottom face and side faces of the cell **12**) in which a blood specimen sample can be in contact with the hemolyzing agent (and which is appropriately determined according to the volume of a blood specimen sample to be measured). The blood processing device **10b** may be introduced into the cell **12** in which the hemolyzing agent is carried after a blood specimen sample has been added, or a blood specimen sample may be added in the cell **12** when the cell **12** of the blood processing device **10b** is introduced into the measurement means. Moreover, a blood specimen sample may be introduced in a cell in a batch manner or in a flow manner.

As has been described in BACKGROUND ART, to measure a hemoglobin, many operation for processing a blood specimen sample are required.

Specifically, to measure HbA_{1c}, an automation apparatus based on the measurement principles on which ion exchange chromatography and latex immunoagglutination are based has been conventionally used. When the amount of the total hemoglobins is about 150 g/l in a sample, the amount is considered to be at a very large for performing measurement with the conventional automation apparatus. Therefore, in general, stages at which complete hemolysis of a blood specimen sample is performed, at which the completely hemolized sample is diluted to have a concentration at which an optimum measurement result can be obtained, and at which the diluted sample is measured based on the measurement principle, are strictly set for the automation apparatus. A1c 2.2 of TOSOH and DLS 2000 of Bayer are examples of typical measurement apparatuses specially designed for measuring HbA_{1c}. The former is based on the ion exchange chromatography principle and the latter is based on latex immunoagglutination. Methods for processing a blood specimen sample used with these apparatuses have a common feature, i.e., a complete hemolysis stage and a dilution stage in each of the methods are automated. Automation apparatuses such as the above described ones have been widely used in the field of clinical examination. However, since various operations are automated, the apparatuses are large and expensive.

As has been described, in measurement of a hemoglobin, if an automation apparatus in which processing operation is incorporated such as the automation apparatuses described above is used, costs become very high. On the other hand, if such an apparatus is not used, examination becomes very complicated because multiple-stage operation is required. Furthermore, assume that a hemoglobin is measured with an immunological method. When complete hemolysis is performed, there may be the cases in which the hemoglobin concentration in a sample becomes very high and thus the sample is considered to be in an antigen excess zone, so that measurement sensitivity is reduced.

However, with the blood processing device **10a** of this embodiment, measurement of components in erythrocytes can be quickly started only by supplying a blood specimen sample to the base material **11** and then setting the base material in the measurement means. Moreover, with the blood processing device **10b** of this embodiment, measurement of components in erythrocytes can be quickly started only by adding a blood specimen sample in the cell **12** and then setting the cell **12** in the measurement means.

### (EMBODIMENT 3)

In this embodiment, a device for measuring a hemoglobin in a blood specimen sample by a measurement method using dry chemistry (which will be herein referred to as a "hemoglobin measurement device") will be described. A hemoglobin measurement device includes a hemolyzing agent of an amount at which a blood specimen sample is partially hemolyzed, a detection substance capable of specifically binding to a hemoglobin and capable of being eluted, and an immobilization substance capable of specifically binding to the hemoglobin.

The "hemolyzing agent of an amount at which a blood specimen sample is partially hemolyzed" in this embodiment means the same as in EMBODIMENT 1.

The detection substance is a substance which is bound to a label and capable of specifically binding to a hemoglobin to be measured. Such labels are well known by persons skilled in the art and include, for example, gold colloid, enzyme (e.g., horseradish peroxidase and glucose oxidase), raidoisotope (e.g., iodine (¹²⁵I, ¹²¹I), carbon (¹⁴C), sulfur (³⁵S), tritium (³H), indium (¹¹²In), technetium (9^{9m}Tc)), a fluorescent label (e.g., fluorescein and rhodamine) and biotin. The label also may be bound to an antibody against a hemoglobin to be measured to form a detection substance. Any antibodies can be used as long as the antibodies is capable of binding to a type of hemoglobin to be measured. The antibody may be a polyclonal antibody or a monoclonal antibody. It is more preferable to use a monoclonal antibody.

The immobilization substance is an arbitrary substance capable of specifically binding to a hemoglobin. If an immunological method is used, a monoclonal antibody against a subject hemoglobin is used as the immobilization substance. It is preferable that the monoclonal antibody does not react with the antibody used as the detection substance but reacts with a different epitope.

In the hemoglobin measurement device of this embodiment, the hemolyzing agent, the detection substance and the immobilization substance are carried in a dry state so that they are arranged in this order from part of the device closer to the blood specimen sample dripping portion of the device.

With the hemoglobin measurement device of this embodiment, therefore, while the blood specimen sample added to the device moves from one layer to another of the layers in which the hemolyzing agent, the detection substance and the immobilization substance are carried, respectively, hemoglobins can be eluted due to hemolysis and also eluted hemoglobins can be detected.

In the hemoglobin measurement device of this embodiment, the layers, for example, are soaked with the hemolyzing agent and then freeze-dried for the purpose of having the agent carried by a base material. The hemolyzing agent reacts with an added blood when being in contact with the blood. Thus, it is preferable that the hemolyzing agent is contained throughout the entire base material so that measurement can be performed without requiring to determine where blood is added in advance. Needless to say, in this case, the amount of the hemolyzing agent contained in the entire base material is a level at which the concentration of eluted hemoglobins in plasma is lower than that in the case of complete hemolysis.

When the blood specimen sample containing eluted hemoglobins moves to the layer in which the detection substance is carried, the detection substance carried in the layer is eluted out, so that eluted hemoglobins specifically react with the eluted detection substance. Thus, the detection substance may be carried in the layer so that the detection substance can be eluted out when encountering a solution, whereas the detection substance is carried in a dry state to form a solid phase in the case where the device is not used. For example, to obtain a layer in which the detection substance is contained in the above-described manner, the detection substance may be soaked in a layer and then freeze-dried.

A hemoglobins bound to the detection substance is bound to the immobilization substance carried in the base material (solid phase matrix). By detecting a specific reaction of the detection substance, a hemoglobin, and the immobilization substance in a sandwich form in the solid phase matrix, the amount of a hemoglobin in the blood specimen sample can be measured. The solid phase matrix where the detection is to be carried out may be made of a matrix material (e.g., cellulose, agarose, dextran and polyethylene) which can be used for a regular immunoassay or the like. Moreover, the solid phase matrix may be a film, a test paper, or a test strip.

The hemoglobin measurement device based on the measurement method using dry chemistry may be a single-layer or multilayer type. Assuming that part of the device closer to a blood specimen sample dripping portion of the device is the upstream, in either of a single-layer type or a multilayer type, the hemolyzing agent, the detection substance and the immobilization substance are carried in this order from the upstream toward the downstream.

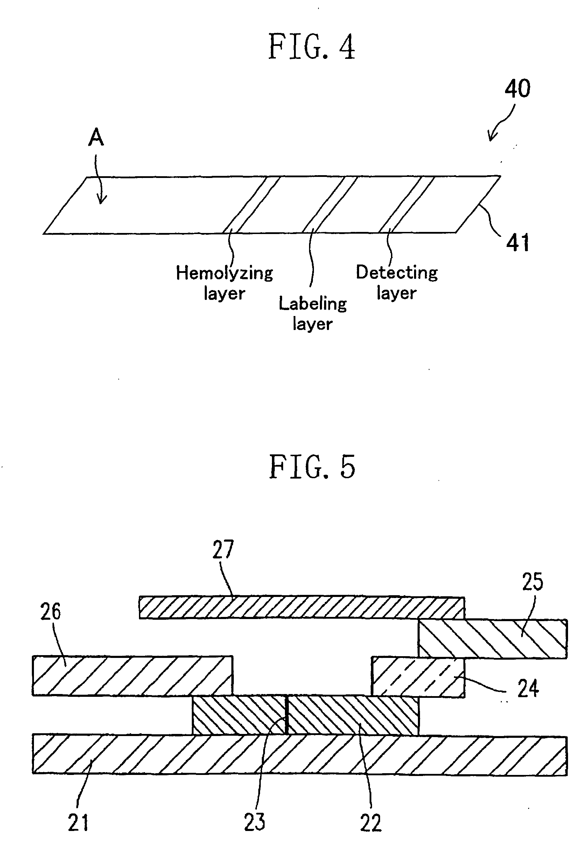

In a singly-layer type device, as shown in FIG. **4**, a layer in which hemolysis of the blood specimen sample is controlled by a hemolyzing agent (which will be herein also referred to as a "hemolysis layer), a layer in which a hemoglobin is labeled with the detection substance (which will be herein also referred to as a "labeling layer"), and a layer in which a hemoglobin bound to an immobilizing reagent is detected (which will be herein also referred to as a "detection layer") are arranged in this order to form a single layer. In the single-layer type device, the hemolyzing agent, the detection substance and the immobilization substance are carried on a base material **41** (solid phase matrix) where the detection is to be carried out so that they are arranged in this order from part of the device closer to a portion **A** of the device where the blood specimen sample is added. The solid phase matrix where the detection is to be carried out may be made of a matrix material (e.g., a nitrocellulose membrane) which can be used for a regular immunoassay or the like. Moreover, the solid phase matrix may be a film, a test paper, or a test strip.

With the arrangement described above, in the step of developing the solid phase matrix, the added blood specimen sample is first brought into contact with the hemolyzing agent, so that blood is partially hemolyzed. Next, a hemoglobin eluted into plasma due to partial hemolysis and the detection substance which can be eluted out specifically bind to each other. Subsequently, a complex of hemoblobin and the detection substance is specifically bound to the immobilization reagent to generate a complex of the detection substance, a hemoglobin and the immobilization substance.

In a multilayer type device, each of a layer in which hemolysis of the blood specimen sample is controlled by a hemolyzing agent, a layer in which a hemoglobin is labeled with the detection substance, and a layer in which a hemoglobin bound to an immobilizing reagent is detected is provided as an individual layer. These layers are stacked so that the blood specimen sample can move from a layer to another. In the multilayer type device, each of the hemolyzing agent, the detection substance and the immobilization substance is carried in an individual layer. The layers containing the hemolyzing agent, the detection substance and the immobilization substance, respectively, are stacked in this order from part of the device closer to the side of the device to which the blood specimen sample is added. The layer in which the hemolyzing agent is carried is made of a material which can soak a liquid added from one surface thereof through to the other surface. For example, the layer in which hemolysis of the blood specimen sample is controlled by a hemolyzing agent is made of a material such as a pulp nonwoven fabric, a mesh cloth (e.g., a gauze cotton cloth) and a glassfiber filter paper. The hemolyzing agent is, for example, soaked in the layer and then freeze-dried, so that the hemolyzing agent is carried in the layer. The detection substance also may be carried in a layer made of a material which is capable of soaking a liquid added from one surface thereof through to the other surface. The detection substance is also soaked in the layer and then freeze-dried, so that the hemolyzing agent is carried in the layer. The immobilization substance may be carried by a solid phase matrix where the detection of a hemoglobin may be carried out. As the solid phase matrix, a nitrocellulose membrane can be used. Immobilization may be performed according procedures of immobilization used in regular immunoassay. These layers may be arranged in contact with each other so that the blood specimen sample can move from a layer to another. With the arrangement described above, the added blood specimen sample is first brought into contact with the hemolyzing agent, so that blood is partially hemolyzed while moving from a layer to another. Next, a hemoglobin eluted into plasma due to the partial hemolysis and the detection substance which is capable being eluted out specifically bind to each other. Subsequently, a complex of a hemoglobin and the detection substance specifically bound to the immobilization reagent to generate a complex of the detection substance, a hemoglobin and the immobilization substance.

The hemoglobin measurement device may also include a layer in which remaining erythrocyte components after the blood specimen sample has been hemolyzed are removed. The layer in which the remaining erythrocyte components are removed may be the same layer in which hemolysis of the blood specimen sample is controlled by a hemolyzing agent. Alternatively, in this device, another layer may be provided as the layer in which remaining erythrocyte components after the blood specimen sample has been hemolyzed are removed, separately from the layer in which hemolysis of the blood specimen sample is controlled by a hemolyzing agent. For example, a layer made of a material such as a pulp nonwoven fabric, a mesh cloth (e.g., a gauze cotton cloth) and a glassfiber filter paper may be used. This layer may absorb a plasma solution containing remaining erythrocyte components which might be unnecessary for a hemoglobin measurement by dry chemistry. Therefore, this layer which will be herein also referred to as an "absorption layer".

An exemplary multilayer type hemoglobin measurement device according to this embodiment will be shown in FIG. **5**. FIG. 5 is a cross-sectional view illustrating the multilayer type hemoglobin measurement device of this embodiment. The multilayer type hemoglobin measurement device may be formed in the structure in which an antibody immobilizing membrane **(22)** on which an antibody **(23)** is immobilized is stacked on a base material **(21)** (the base material may be polyethylene terephthalate (PET)), a labeled antibody freeze-dry carrier layer **(24)** is stacked and then a hemolyzing agent carrier layer **(25)** is stacked thereon. In this case, as shown in FIG. **2**, the hemolyzing agent carrier layer **(25)**, the labeled antibody freeze-dry carrier layer **(24)** and the antibody immobilizing membrane **(22)** may be arranged so that each of the hemolyzing agent carrier layer **(25)**, the labeled antibody freeze-dry carrier layer **(24)** and the antibody immobilizing membrane **(22)** partially overlaps with a layer which is located immediately thereunder. With an overlapping part of each of the layers and the membrane, the blood specimen sample moves from a layer to another. Accordingly, the blood specimen sample is hemolyzed and is bound to a labeled antibody and an immobilized antibody. An antibody capable of specifically binding to a hemoglobin is adhered to an exposed region of the antibody immobilizing membrane **(22)** (i.e., a region thereof overlapping with neither of the hemolyzing agent carrier layer **(25)** nor the labeled antibody freeze-dry carrier layer **(24)**), and a reaction of the hemoglobin in the blood specimen sample can be detected in the region. Moreover, the absorption layer **(26)** is stacked so as to overlap with part of the antibody immobilizing membrane **(22)** located on the opposite side to a side thereof to which the blood specimen sample is added (i.e., the side of the antibody immobilizing membrane which does not overlap with the labeled antibody freeze-dry carrier layer (**24**)). The absorption layer can absorb the plasma solution containing remaining erythrocyte membranes which exist in a reaction detection region of the device and might be unnecessary for detection, thereby assisting detection of the reaction. Furthermore, part of the device extending from a region of the device in which the three layers, i.e., the hemolyzing agent carrier layer **(25)**, the labeled antibody freeze-dry carrier layer **(24)** and the antibody immobilizing membrane **(22)**, are overlapping to the reaction detection region may be covered with an impermeable sheet **(27)** in order to keep a moist condition created by an added sample from drying up and also keep a substance which might hinder a measurement of a sample from being mixed into the sample. As such an impermeable sheet, cellophane may be used. Other part of the hemolyzing agent carrier layer **(25)** than the overlapping part thereof is not covered with the impermeable sheet. The blood specimen sample may be added to the uncovered part of the hemolyzing agent carrier layer **(25)**. The impermeable sheet **(27)** may also cover part of the absorption layer **(26)** overlapping with the antibody immobilizing membrane **(22)**, and at least part of a region of the absorption layer **(26)** which does not overlap with the antibody immobilizing membrane **(22)**. With the covering, absorption of the plasma from the antibody immobilizing membrane **(22)** into the absorption layer **(26)** can be prevented from being hindered.

As has been descried, if the blood specimen sample is partially hemolyzed in measuring components in hemocytes, serial dilution required in a known method can be removed. In the case of manual operation, if the method of the present invention is used, dilution operation can be omitted. Moreover, in the case in which the serial dilution is automated, the serial dilution can be removed the automation apparatus, resulting in reduction in the size and costs of the apparatus.

Therefore, the present invention provides a blood processing method, a hemoglobin measurement method, a blood processing device and a hemoglobin measurement device which allow measurements of components such as hemoglobins in erythrocytes to be performed in a more simple manner. According to the present invention, a hemoglobin can be quickly measured in a more simple manner.

### Examples

Next, examples of the present invention will be described based on measurement examples of HbA_{1c}. Note that the scope of the present invention is no way limited by the following examples. Each of materials and reagents used in the examples is available from a commercial supply source unless any other specific description is given.

### (Example 1: Appropriate range for the amount of hemolyzing agent to be added in absorptiometry)

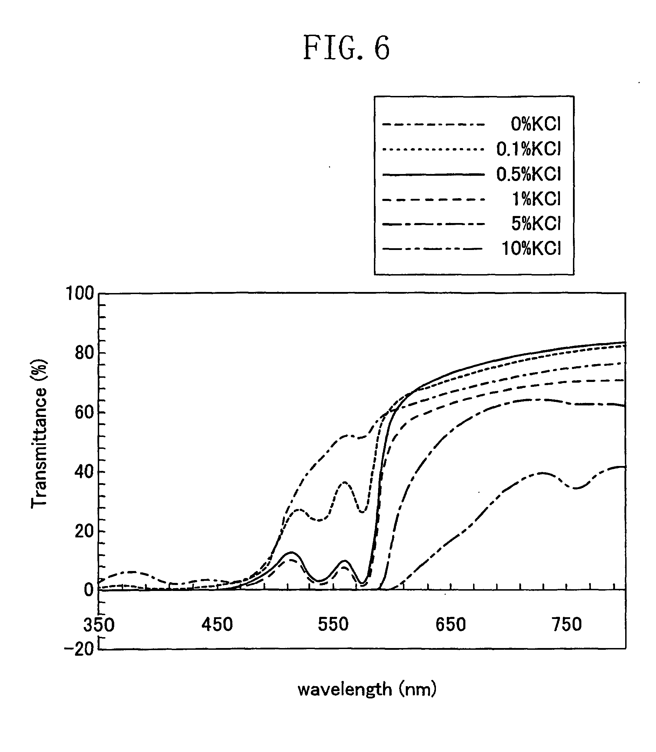

1.5 ml of a whole blood specimen sample (4.8% HbA1c value) was added to each of potassium chlorides of 1.5 mg, 7.5 mg. 15 mg, 75 mg and 150 mg (i.e., the percentages of the whole blood specimen sample in obtained mixture solutions were 0.1%, 0.5%, 1%, 5%, and 10% (weight/volume), respectively). The mixture solutions were made turbid and centrifuged to obtain a 1.0 ml supernatant fluid of each of the mixture solutions. Light transmittance of each of the obtained supernatant fluids with respect to light in a wavelength range from 350 to 800 nm was examined. The examination results are shown in FIG. **6**. FIG. **6** shows that, for example, the appropriate amount of an added hemolyzing agent (KCl in this example) was about 0.1-0.5% at a wavelength of 500 nm and about 0.1-10% at a wavelength of 700 nm.

### (Example 2: Relationships between the amount of hemolyzing agent, and Hb concentration and HbA_{1c} value)

1.5 ml of a whole blood specimen sample (4.8% HbA1c value) was added to each of potassium chlorides of 1.5 mg, 7.5 mg. 15 mg, 75 mg and 150 mg (i.e., the percentages of the whole blood specimen sample in obtained mixture solutions were 0.1%, 0.5%, 1%, 5% (weight/volume), and 10%, respectively). The mixture solutions were made turbid and centrifuged to obtain a 1.0 ml supernatant fluid of each of the mixture solutions. The concentration of Hb existing in each supernatant fluid was measured by a SLS-hemoglobin method. The SLS-hemoglobin method will be described. The SLS-hemoglobin method was performed using Hemoglobin B-Test Wako commercially available from Wako Pure Chemical Industries, Ltd. A 0.02 ml hemoglobin standard solution (1 g/l, 5 g/l, 10 g/l, 50 g/l, 100 g/l or 150 g/l) was added to 0.67 mM lauric acid sodium of 5.0 ml, mixed well, and then left to stand at room temperature for about 3 minutes to prepare a hemoglobin control solution. For hemoglobin control solutions prepared in the above-described manner, absorbance was measured at a wavelength of 540 nm. Using obtained values, a standard curve for absorbance (at a wavelength of 540 nm) with respect to hemoglobin concentration was made. Next, as in the same manner, absorbance was measured for samples having been processed at different hemolysis percentages using 0.02 ml of each of the above-described supernatant fluids. Using the standard curve, hemoglobin concentration was calculated for each of the samples.

On the other hand, HbA_{1c} values were measured using HPLC (A1c2.2, TOSOH), specially designed for measuding HbA_{1c}. Each of hemolysis-processed samples was put into a test tube and set in the measurement apparatus to operate automatic measurement. An obtained HbA_{1c} value here corresponds to the ratio (%) of HbA_{1c} to the amount of total hemoglobins.

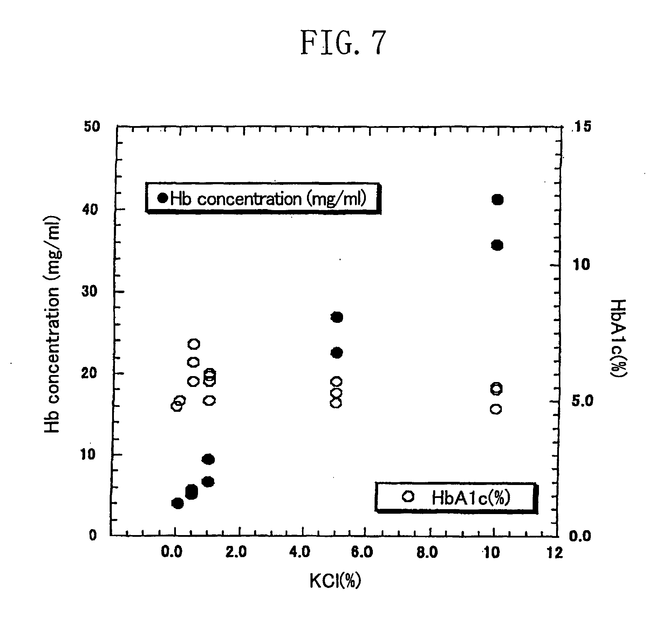

Results obtained from the measurement are shown in FIG. **7**. In FIG. **7**, the bottom abscissa indicates the concentration (%) of added potassium sodium, the left ordinate indicates Hb concentration (mg/ml) and the right ordinate indicates HbA_{1c} values (%). Moreover, the block circles in FIG. **7** indicate results of the Hb concentration and the white circles indicate results of the HbA_{1c} value.

FIG. **7** shows that the concentration of Hb eluted due to hemolysis was increased depending on the KCl concentration. Furthermore, FIG. **7** shows that while the Hb concentration was varied depending on the amount of the hemolyzing agent, HbA_{1c} was not varied. Therefore, it has been suggested that there is no need for complete hemolysis in measuring the ratio (%) of a hemoglobin to the amount of the total hemoglobins and thus serial dilution does not have to be performed.

### (Example 3: Measurement of HbA1c by immunochromatography)

### (Formation of Hb measurement device and HbA_{1c} measurement device)

In this example, a device usable for measuring Hb was formed. As shown in FIG. **5**, this device is a multilayer type device in which hemolysis with each of a hemolyzing agent, a reaction with an antibody and detection of the reaction takes place in individual layers, respectively. FIG. **5** is a cross-sectional view illustrating an exemplary multilayer device according to the present invention. First, process steps for forming each layer will be described.

### < Gold-colloid-labeled antibody freeze-dry immobilization substance (24)>

198 ml of distilled water and 2 ml of a 1% gold chloride solution were put together into an Erlenmeyer flask. The mixture solution was boiled and then 4 ml of a 1% sodium citrate was quickly added into the boiled mixture solution. After the sodium citrate having been added, the color change of the mixture solution into red purple was observed and then the mixture solution was left to stand in the boiling state for 15 minutes. Thereafter, the Erlenmeyer flask was taken out and left at room temperature to be naturally cooled down. Thus, a gold colloid solution was obtained. 0.2 M of a potassium carbonate solution was added to the obtained gold colloid solution to adjust the colloid solution to pH 8.9. 1.6 ml of a 1.55 mg/ml solution of an anti-Hb monoclonal antibody produced from a cell strain of No. FERM BP-7288 deposited in the International Patent Organism depositary, National Institute of Advanced Industries Science and Technology was added to the gold colloid solution and then the colloid solution was stirred for 3 minutes. Furthermore, 20 ml of a 10% bovine plasma albumin (BSA) solution (pH 8.9) was added to the gold colloid solution and stirred for another 3 minutes. Thereafter, to remove unlabeled antibodies and BSA, the colloid solution was centrifuged and a supernatant fluid was removed by an aspirator. Then, for cleaning, the colloid solution was suspended again using a 1% bovine plasma albumin saline solution (BSA·PBS). The colloid solution was again centrifuged and a supernatant fluid was removed by an aspirator. The gold-colloid-labeled anti-Hb monoclonal antibody 1% BSA·PBS suspension obtained in the above-described manner was filtered with a 0.45 µm filter to remove aggregates. Thereafter, a gold-colloid-labeled anti-Hb antibody **21** suspension was impregnated with a glass fiber filter paper, was frozen using liquid nitrogen, and then was left to stand for 24 hours. In this manner, a freeze-dry immobilization substance was formed.

### <Hb measurement/antibody immobilizing membrane (22)>

A solution (PBS) of an anti-Hb monoclonal antibody produced from a cell strain of No. FERM BP-7289 deposited in the International Patent Organism depositary, National Institute of Advanced Industries Science and Technology was immobilized on a nitrocellulose membrane. After the immobilization, blocking was performed with skimmed milk. Subsequently, the immobilized antibody was washed twice with 0.1M tris (pH 8.2) and dried.

### <HbA_{1c} measurement/antibody immobilizing membrane (22)>

A solution of an anti-HbAlc monoclonal antibody produced from a cell strain of No. FERM BP-7636 deposited in the International Patent Organism depositary, National Institute of Advanced Industries Science and Technology was immobilized on a nitrocellulose membrane. After the immobilization, blocking was performed with skimmed milk. Subsequently, the immobilized antibody was washed twice with 0.1M tris (pH 8.2) and dried.

### <Hemolyzing agent carrier solid phase (25)>

0.5% (w/v) KCl to the total volume of a sample was impregnated with Reed Cooking Paper™ (commercially available from Lion) and was freeze-dried.

### <Construction of Hb measurement device and HbA_{1c} measurement device>

As shown in FIG. **5,** a Hb measurement device and a HbA_{1c} measurement device were constructed with the gold-colloid-labeled antibody freeze-dry carrier layer, the Hb measurement membrane or the HbA_{1c} measurement membrane, and the hemolyzing agent carrier layer, which have been formed in the above described manners. Construction process steps for this device will be described.

An antibody immobilizing membrane **(22)** on which an antibody **(23)** was immobilized was stacked on a base material **(21)** (polyethylene terephthalate: Matsumoto Production), a gold-colloid-labeled antibody freeze-dry carrier layer **(24)** was stacked and then a hemolyzing agent carrier layer **(25)** was stacked thereon. In this case, as shown in FIG. **5**, the hemolyzing agent carrier layer **(25)**, the gold-colloid-labeled antibody freeze-dry carrier layer **(24)** and the antibody immobilizing membrane **(22)** were arranged so that each of the hemolyzing agent carrier layer **(25)**, the labeled antibody freeze-dry carrier layer **(24)** and the antibody immobilizing membrane **(22)** partially overlap with a layer which was located immediately thereunder. An antibody capable of specifically binding to a hemoglobin was adhered in an exposed region of the antibody immobilizing membrane **(22)** (i.e., a region thereof overlapping with neither of the hemolyzing agent carrier layer **(25)** nor the gold-colloid-labeled antibody freeze-dry carrier layer **(24)**). This region was determined to be a region in which a reaction of a hemoglobin in the blood specimen sample was to be detected. Moreover, a glassfiber filter paper absorption layer **(26)** was stacked so as to overlap with part of the antibody immobilizing membrane **(22)** located on the opposite side to a side thereof to which the blood specimen sample was added (i.e., the side of the antibody immobilizing membrane not overlapping with the gold-colloid-labeled antibody freeze-dry carrier layer **(24)**). Furthermore, part of the device extending from a region of the device in which the three layers, i.e., the hemolyzing agent carrier layer **(25)**, the gold-colloid-labeled antibody freeze-dry carrier layer **(24)** and the antibody immobilizing membrane **(22)**, were overlapping to the reaction detection region was covered with an impermeable sheet **(27)** (cellophane). Other part of the hemolyzing agent carrier layer **(25)** than the overlapping part thereof was exposed. This exposed part was determined to be a part to which a blood specimen sample was to be dripped. Part of the device part of the glassfiber filter paper absorption layer **(26)** overlapping with the antibody immobilizing membrane **(22)** and at least part of a region of the glassfiber filter paper absorption layer **(26)** not overlapping with the antibody immobilizing membrane **(22)** were also covered with the impermeable sheet **(27).** The above-described covering was made in order to keep absorption of the plasma from the antibody immobilizing membrane **(22)** into the glassfiber filter paper absorption layer **(26)** from being hindered.

### <Measurement of a hemoglobin using Hb measurement device and HbA_{1c} measurement device>

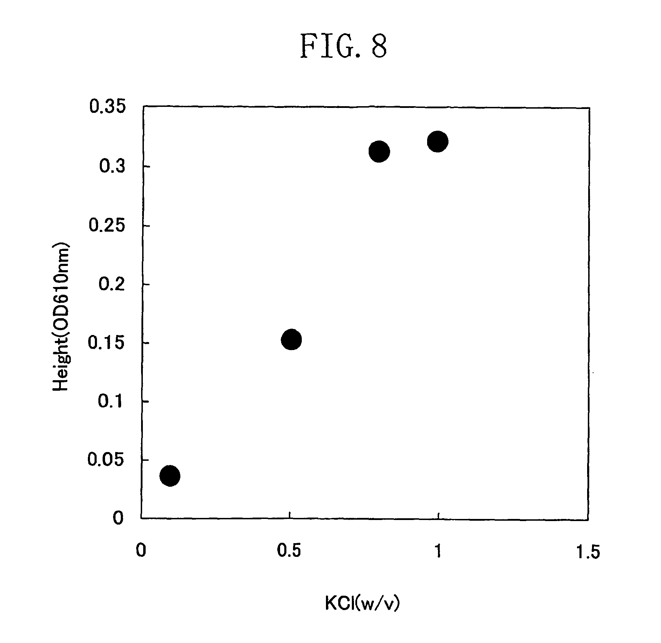

160 µl of blood was dripped to each of devices including hemolyzing agent carrier layers in which 0%, 0.05%, 0.1%, 0.5%, 0.7%, 1.0%, and 3.0% of KCl (w/v) were carried, respectively, and after five minutes after the dripping, the reflection absorption at a wavelength of 610 nm of each of the layers was measured. Measurement results are shown in FIG. **8**.

FIG. **8** shows that color intensities appear from 0.1% to 1.0% (w/v) KCls and therefore an optimum range of the KCl amount is from 0.1% to 1.0% (w/v). In this device, 0.5% (w/v) KCl which is the middle of the above-described range is preferable. As has been described in <Hemolyzing agent carrier solid phase **(25)**>, in this example, 0.5% (w/v) KCl was carried by the hemolyzing agent carrier solid phase **(25)** to perform a measurement of HbA_{1c}.

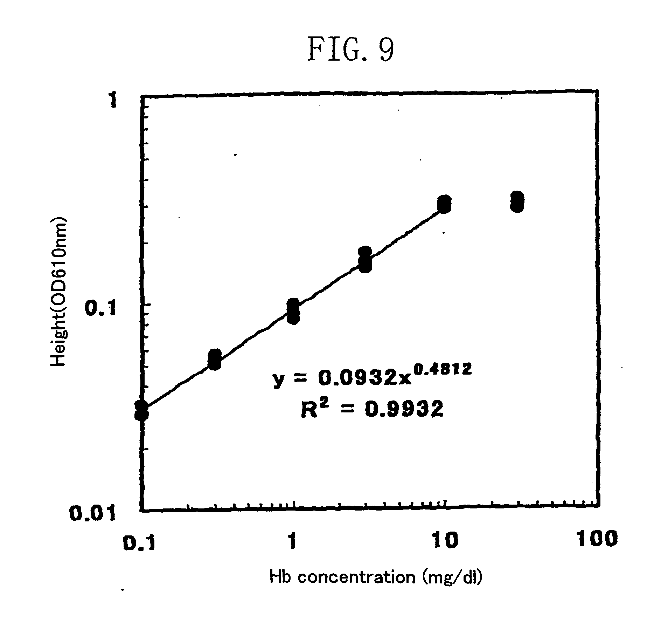

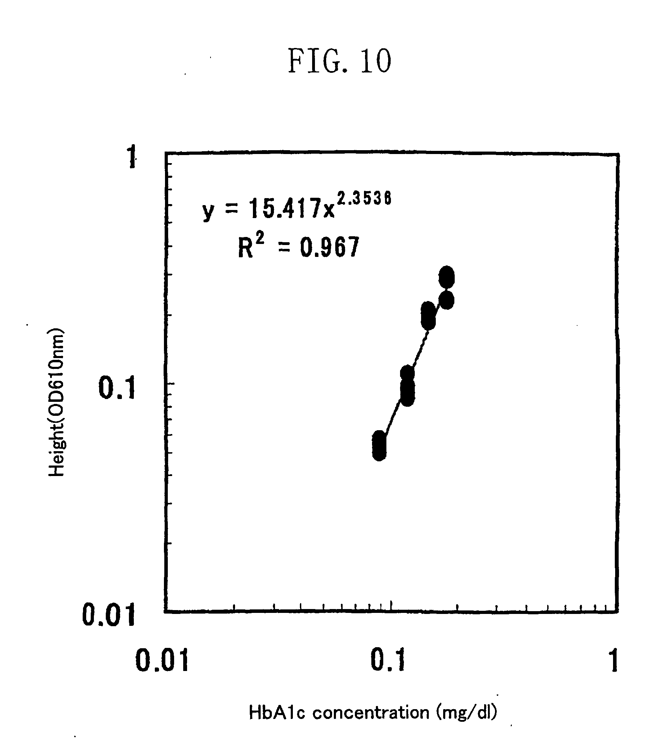

First, a series of dilutions were prepared using a Hb standard solution or a HbA_{1c} standard solution. 160 µL of each of the dilutions was dripped to a device. Five minutes after the dripping, the reflection absorbance (at a wavelength of 610 nm) was measured and a standard curve was made. The standard curve of the Hb measurement device is shown in FIG. **9** and the standard curve of the HbA_{1c} measurement device is shown in FIG. **10.** In FIG. **9,** the abscissa indicates Hb concentraion (mg/dl) and the ordinate indicates reflection absorbance values at a wavelength of 610 nm. Moreover, the block circles indicate obtained absorbance results for the dilutions having different Hb concentrations. In FIG. **10,** the abscissa indicates HbA_{1c} concentration (mg/dl) and the ordinate indicates reflection absorbance values at a wavelength of 610 nm. Moreover, the block circles indicate obtained absorbance results for the dilutions having different HbA_{1c} concentrations.

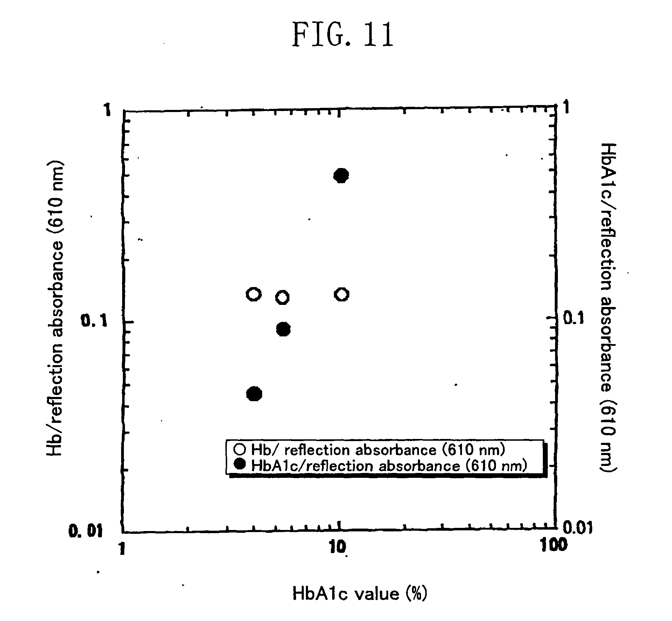

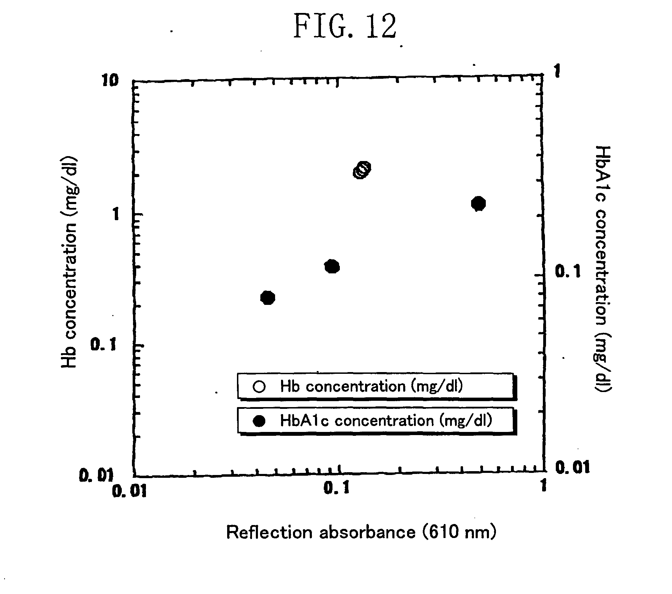

Next, 160 µl of each of blood specimen samples containing 4.0, 5.4 and 10.4% HbA_{1c}, respectively, was dripped to a device, and five minutes after the dripping, reflection absorbance was measured. Measurement results are shown in FIG. **11.** In FIG. **11,** the abscissa indicates a HbA_{1c} value (%), the left ordinate indicates reflection absorbance with respect to Hb, and the right ordinate indicates reflection absorbance with respect to HbA_{1c}. Moreover, the black circles indicate results for Hb concentration and the white circles indicate results for HbA_{1c}. Each absorbance value of the results shown in FIG. **11** was revolved from the standard curve of FIG. **9** or FIG. **10** to obtain the Hb concentration and the HbA_{1c} concentration (FIG. **12**). In FIG. **12,** the abscissa indicates reflection absorbance at a wavelength of 610 nm and the left ordinate indicates Hb concentration (mg/dl) obtained by calculation, and the right ordinate indicates HbA_{1c} concentration (mg/dl) obtained by calculation. Moreover, the block circles in FIG. **12** indicate results for the Hb concentration and the white circles indicate results for the HbA_{1c} concentration (mg/dl). The Hb concentrations of the above-described blood specimen samples were almost the same. This fact shows that each of the blood specimen samples was hemolyzed to almost the same level. Using the Hb concentration (mg/dl) and the HbA_{1c} concentration (mg/dl) obtained in measurement with the device, a HbA_{1c} value (%) was determined for each of the samples having different HbA_{1c} value (by calculating based on HbA_{1c} concentration (mg/dl) / Hb concentration (mg/dl) x 100). As a result, 3.9%, 5.7%, and 11.2% were obtained for the specimen having a HbA_{1c} value of 4.0%, the specimen having a HbA_{1c} value of 5.4%, and the specimen having a HbA_{1c} value of 10.4%, respectively. From this calculation result, it was confirmed that by controlling hemolysis so that a blood specimen sample is partially hemolyzed, a hemoglobin can be measured.

The results described above show that a device which does not require a series of dilutions and allow measurement in a simple manner with a single stage dilution was obtained.

According to the present invention, a hemoglobin can be quickly measured in a more simple manner.

### INDUSTRIAL APPLICABILITY

The present invention is useful in the areas of nutrition control and medical diagnosis.

## Claims

1. A blood processing method comprising the steps of:
(a) preparing a blood specimen sample containing erythrocytes, and a hemolyzing agent; and
(b) mixing the blood specimen sample and the hemolyzing agent to prepare a mixture sample
wherein the hemolyzing agent is a solid reagent for destroying erythrocyte membranes.

2. The blood processing method of claim 1, wherein in the step (b), hemocyte components remain in the mixture sample.

3. The blood processing method of claim 1, wherein the hemolyzing agent is a reagent for changing osmotic pressure against erythrocyte membranes.

4. The blood processing method of claim 3, wherein the hemolyzing agent is an inorganic salt.

5. The blood processing method of claim 4, wherein the concentration of the inorganic salt in the mixture sample is in a range of 0.05-10% (w/v).

6. A hemoglobin measurement method comprising the steps of:
(a) preparing a blood specimen sample containing erythrocytes, and a hemolyzing agent;
(b) mixing the blood specimen sample and the hemolyzing agent to prepare a mixture sample; and
(c) measuring a hemoglobin contained in plasma in the mixture sample
wherein the hemolyzing agent is a solid reagent for destroying erythrocyte membranes.

7. The hemoglobin measurement method of claim 6, wherein in the step (c), as the hemoglobin measurement method for measuring a hemoglobin contained in plasma in the mixture sample, any one of an immunochromatography, an immunoconcentration assay and a solid phase enzyme linked immunoassay, a colorimetry slide and an electrode method slide is used.

8. The hemoglobin measurement method of claim 6, wherein in the step (c), at least two hemoglobins are measured.

9. The hemoglobin measurement method of claim 8, wherein said at least two hemoglobins are the total hemoglobins and HbA1c.

10. The hemoglobin measurement method of claim 6, wherein the hemoglobin is a glycated hemoglobin.

11. A blood processing device comprising:
a base material; and
a solid hemolyzing agent for destroying erythrocyte membranes
wherein the hemolyzing agent is carried on the base material at an amount at which when a mixture sample of a blood specimen sample containing erythrocytes and the hemolyzing agent is formed, hemocyte components remain in the mixture sample.

12. A hemoglobin measurement device for measuring a hemoglobin in a blood specimen sample containing erythrocytes, comprising:
a base material having a sample adding portion to which the blood specimen sample is added;
a hemolyzing agent for destroying erythrocyte membranes;
a detection substance capable of specifically binding to a hemoglobin; and
an immobilization substance capable of specifically binding to the hemoglobin
wherin the hemolyzing agent is carried on the base material at an amount at which when a mixture sample of a blood specimen sample containing erythrocytes and the hemolyzing agent is formed, hemocyte components remain in the mixture sample,
wherein the hemolyzing agent, the detection substance and the immobilization substance are individually carried in a solid form on the base material, and
wherein the hemolyzing agent, the detection substance and the immobilization substance are arranged in this order from part of the base material closer to a sample adding portion to which the blood specimen sample is added.

13. A hemoglobin measurement device for measuring a hemoglobin in a blood specimen sample containing erythrocytes, comprising:
a substrate;
a sample adding portion which is provided on the base material and to which the blood specimen sample is added;
a hemolyzing layer which is provided on the base material, contains a solid hemolyzing agent for destroying erythrocyte membranes, and is for controlling hemolysis of the blood specimen sample;
a labeling layer which is provided on the base material, contains a detection substance capable of specifically binding to a hemoglobin, and is for labeling the hemoglobin; and
a detecting layer which is provided on the base material, contains an immobilization reagent capable of specifically binding to a hemoglobin, and is for detecting the hemoglobin
wherein the blood specimen sample can move between the hemolyzing layer, the labeling layer and the detecting layer.

14. The hemoglobin measurement device of claim 13, wherein the hemoglobin measurement device further includes a layer for removing remaining erythrocyte components after the blood specimen sample has been hemolyzed.

15. The hemoglobin measurement device of claim 13, wherein the hemolyzing layer, the labeling layer and the detecting layer are arranged in a single layer.

1. (Amended) A blood processing method comprising the steps of:
(a) preparing a blood specimen sample containing erythrocytes, and a hemolyzing agent; and
(b) mixing the blood specimen sample and the hemolyzing agent to prepare a mixture sample
wherein the hemolyzing agent is a solid reagent for destroying erythrocyte membranes, and
wherein in the step (b), hemocyte components remain in the mixture sample.

2. (Cancelled)

3. The blood processing method of claim 1, wherein the hemolyzing agent is a reagent for changing osmotic pressure against erythrocyte membranes.

4. The blood processing method of claim 3, wherein the hemolyzing agent is an inorganic salt.

5. blood processing method of claim 4, wherein the concentration of the inorganic salt in the mixture sample is in a range of 0.05-10% (w/v).

6. (Amended) A hemoglobin measurement method comprising the steps of:
(a) preparing a blood specimen sample containing erythrocytes, and a hemolyzing agent;
(b) mixing the blood specimen sample and the hemolyzing agent to prepare a mixture sample; and
(c) measuring a hemoglobin contained in plasma in the mixture sample
wherein the hemolyzing agent is a solid reagent for destroying erythrocyte membranes, and
wherein in the step (b), hemocyte components remain in the mixture sample.

7. The hemoglobin measurement method of claim 6, wherein in the step (c), as the hemoglobin measurement method for measuring a hemoglobin contained in plasma in the mixture sample, any one of an immunochromatography, an immunoconcentration assay and a solid phase enzyme linked immunoassay, **a colorimetry slide method** and **an** **electrode slide method** is used.

8. The hemoglobin measurement method of claim 6, wherein in the step (c), at least two hemoglobins are measured.

9. The hemoglobin measurement method of claim 8, wherein said at least two hemoglobins are the total hemoglobins and HbA1c.

10. The hemoglobin measurement method of claim 6, wherein the hemoglobin is a glycated hemoglobin.

11. A blood processing device comprising:
a base material; and
a solid hemolyzing agent for destroying erythrocyte membranes
wherein the hemolyzing agent is carried on the base material at an amount at which when a mixture sample of a blood specimen sample containing erythrocytes and the hemolyzing agent is formed, hemocyte components remain in the mixture sample.

12. A hemoglobin measurement device for measuring a hemoglobin in a blood specimen sample containing erythrocytes, comprising:
a base material having a sample adding portion to which the blood specimen sample is added;
a hemolyzing agent for destroying erythrocyte membranes;
a detection substance capable of specifically binding to a hemoglobin; and
an immobilization substance capable of specifically binding to the hemoglobin
wherin the hemolyzing agent is carried on the base material at an amount at which when a mixture sample of a blood specimen sample containing erythrocytes and the hemolyzing agent is formed, hemocyte components remain in the mixture sample,
wherein the hemolyzing agent, the detection substance and the immobilization substance are individually carried in a solid form on the base material, and
wherein the hemolyzing agent, the detection substance and the immobilization substance are arranged in this order from part of the base material closer to a sample adding portion to which the blood specimen sample is added.

13. A hemoglobin measurement device for measuring a hemoglobin in a blood specimen sample containing erythrocytes, comprising:
a substrate;
a sample adding portion which is provided on the base material and to which the blood specimen sample is added;
a hemolyzing layer which is provided on the base material, contains a solid hemolyzing agent for destroying erythrocyte membranes, and is for controlling hemolysis of the blood specimen sample;
a labeling layer which is provided on the base material, contains a detection substance capable of specifically binding to a hemoglobin, and is for labeling the hemoglobin; and
a detecting layer which is provided on the base material, contains an immobilization reagent capable of specifically binding to a hemoglobin, and is for detecting the hemoglobin
wherein the blood specimen sample can move between the hemolyzing layer, the labeling layer and the detecting layer.

14. The hemoglobin measurement device of claim 13, wherein the hemoglobin measurement device further includes a layer for removing remaining erythrocyte components after the blood specimen sample has been hemolyzed.

15. The hemoglobin measurement device of claim 13, wherein the hemolyzing layer, the labeling layer and the detecting layer are arranged in a single layer.
